Europäisches Patentamt

European Patent Office

Office européen des brevets

(11) Veröffentlichungsnummer: **0 000 774**
**B1**

# EUROPÄISCHE PATENTSCHRIFT

(21) Anmeldenummer: 78100586.3

(22) Anmeldetag: 03.08.78

(51) Int. Cl³: **A 61 K 31/505, //**
**(A 61 K 31/505, 31/415)**

(54) Antithrombotische Arzneimittelkombination und Verfahren zu ihrer Herstellung

(30) Priorität: 09.08.77 DE 2735830

(43) Veröffentlichungstag der Anmeldung:
21.02.79 Patentblatt 79/04

(45) Bekanntmachung des Hinweises auf die Patenterteilung:
28.05.80 Patentblatt 80/11

(84) Benannte Vertragsstaaten: BE CH DE FR GB LU NL
SE

(56) Entgegenhaltungen:
Chemical Abstracts, 85, 56457b (1976)

(73) Patentinhaber: **Dr. Karl Thomae GmbH**
**Postfach 720**
**D - 7950 Biberach (Riss) (DE)**

(72) Erfinder: **Schröter, Hans Wolfgang, Dr.**
**Johann-Sebastian-Bach-Strasse 22**
**D - 7950 Biberach 1 (DE)**
**Haarmann, Walter, Dr.**
**Schlierholzweg 8**
**D - 7950 Biberach 1 (DE)**
**Roch, Josef, Dr. Dipl.-Chemiker**
**Stecherweg 19**
**D - 7950 Biberach 1 (DE)**
**Harker, Laurence A., Prof.**
**Harborview Medical Center**
**325 Ninth Avenue**
**Seattle, Washington 98104 (US)**

Courier Press, Leamington Spa, England.

# 0 000 774

Antithrombotische Arzneimittelkombination und Verfahren zu ihrer Herstellung

Die Erfindung betrifft eine neue antithrombotisch wirkende Arzneimittelkombination sowie ein Verfahren zu ihrer Herstellung. 2,6-Bis(diäthanolamino)-4,8-dipiperidino-pyrimido[5,4-d]pyrimidin (generic name: DIPYRIDAMOL) wird bereits seit langem als koronarerweiterndes Heilmittel verwendet. Es wurde erstmals im bristischen Patent 807 826 beschrieben. Ebenso ist 1,2-Diphenyl-3,5-dioxo-4-(2-phenylsulfinyläthyl)-pyrazolidin (generic name: SULFINPYRAZON) bereits seit langem als Antigichtmittel im Handel, es wurde erstmals in der Helv. chim. Acta *44*, 236 (1961) beschrieben. Bei beiden Heilmitteln wurde in der Folgezeit auch eine gute antithrombotische Wirkung gefunden, siehe beispielsweise Therapiewoche *26*, 8464—8489 (1976).

Bei beiden Substanzen ist jedoch zur Erzielung einer antithrombotischen Wirkung eine relativ hohe Dosis erforderlich, wobei sich schon die Nebenwirkungen beider Verbindungen bemerkbar machen; beim Dipyridamol sind es gewisse Kreislaufwirkungen, die sich in Kopfschmerzen äußern, beim Sulfinpyrazon sind es Magenschmerzen wegen der in hohen Dosen ulcerogenen Wirkung dieser Substanz.

Überraschenderweise wurde nun eine starke synergistische Wirkung der beiden Wirkstoffe festgestellt. Bei einer Kombination von Dipyridamol und Sulfinpyrazon können die erforderlichen Dosen zur Erreichung des gleichen antithrombotischen Effektes wesentlich gesenkt werden. Diese synergistische Wirkung wurde im folgenden Versuchsmodell an Pavianen festgestellt:

Männlichen Pavianen mit einem Gewicht von 8—12 kg wird unter Narkose ein A—V-Shunt zwischen Arteria und Vena femoralis angelegt. Dieser Shunt ist ca. 50 cm lang und besteht aus einem besonders präparierten Silastikschlauch. Durch den Fremdkörperreiz dieser körperfremden Oberfläche kommt es zu einem erhöhten Thrombozytenverbrauch (da der Organismus bestrebt ist, diesen "Defekt" abzudecken). Dieser läßt sich leicht messen, indem man dem Versuchstier mit Chrom 51 radioaktiv markierte Thrombozyten injiziert und deren Verschwinden aus der Blutbahn durch 2 x tägl. Messen verfolgt. Bei einem normalen Thrombozytenumsatz beträgt die Lebensdauer der Thrombozyten etwa 5 Tage, bei Tieren mit einem eingesetzten Shunt verkürzt sich die mittlere Thrombozytenlebenszeit (als Ausdruck eines erhöhten, Thrombozytenumsatzes) auf etwa 2—2,5 Tage.

Bei der Substanzprüfung wird die zu prüfende Substanz über den Versuchszeitraum von 4—5 Tagen den wachen Tieren (meist) 4 mal täglich oral verabreicht.

Die bei dieser Methode erzielten Ergebnisse sind in der nachstehenden Tabelle wiedergegeben:

Wirkung der Kombination von DIPYRIDAMOL mit SULFINPYRAZON bei Pavianen

Überlebenszeit der Thrombozyten (in Tagen)

| Tier | Kontrolle | Ausgangswert des Tiers mit eingelegtem Shunt | DIPYRIDAMOL | | SULFINPYRAZON | | Kombination von Dipyridamol mit Sulfinpyrazon |
|---|---|---|---|---|---|---|---|
| | | | 2,5 mg/kg/ Tag | 10 mg/kg/ Tag | 2,5 mg/kg/ Tag | 100 mg/kg/ Tag | je 2,5 mg/kg/ Tag |
| 1 | 5,5 | 2,0 | 2,4 | 5,1 | 1,9 | 5,3 | 4,9 |
| 2 | 5,1 | 2,7 | 2,6 | 4,9 | 2,8 | 4,7 | 5,3 |
| 3 | 5,4 | 2,3 | 2,9 | 5,6 | 2,7 | 5,0 | 5,1 |
| 4 | 5,6 | 2,9 | 3,3 | 5,3 | 2,4 | 5,3 | 5,0 |
| 5 | 5,2 | 2,5 | 3,1 | 5,0 | 2,9 | 4,9 | 4,7 |
| Mittelwert | 5,4±0,2 | 2,5±0,4 | 2,9±0,4 $p<0,05$ | 5,2±0,3 $p<0,001$ | 2,5±0,4 $p>0,50$ | 5,0±0,3 $p<0,001$ | 5,0±0,2 $p<0,001$ |

Aus der Tabelle geht klar hervor, daß für die Normalisierung der verkürzten Thrombozytenlebenszeit 10 mg/kg/Tag Dipyridamol oder 100 mg/kg/Tag Sulfinpyrazon erforderlich ist, daß jedoch mit einer Kombination von je 2,5 mg/kg/Tag der beiden Wirkstoffe der gleiche Effekt erreicht wird. Es ist bekannt, daß sich die Ergebnisse über die Normalisierung der Thrombozytenlebenszeit sehr gut vom Affen auf den Menschen übertragen lassen, beispielsweise ist beim Menschen zur Erzielung des gleichen Effektes die gleiche Dosis von Dipyridamol pro Kilogramm erforderlich, wie beim Affen. Die

# 0 000 774

Normalisierung der Thrombozytenlebenszeit ist therapeutisch von sehr großer Bedeutung, da die beim Menschen auftretende Verkürzung der Thrombozytenlebenszeit ein Indiz für eine Neigung zu Thrombosen ist. Die neue Arzneimittelkombination ist daher zur Vorbeugung und Heilung von thromboembolischen Krankheiten gut geeignet.

Eine Einzeldosis für Erwachsene enthält zwischen 25 mg und 75 mg der beiden Wirkstoffe, vorzugsweise 50 mg Dipyridamol und 50 mg Sulfinpyrazon; die Einzeldosis wird vorzugsweise 3 mal täglich verabreicht, die mittlere Tagesdosis beträgt somit 150 mg/kg von jedem der beiden Wirkstoffe. Da beide Wirkstoffe schon seit vielen Jahren im Handel sind, brauchen keine Angaben über die Toxizität gemacht werden, da diese in beiden Fällen gering ist.

Gegenstand der Erfindung ist des weiteren ein Verfahren zur Herstellung der erfindungsgemäßen Arzneimittelkombination, welches dadurch gekennzeichnet ist, daß 2,6-Bis(diäthanolamino)-4,8-dipiperidino-pyrimido[5,4-d]pyrimidin und 1,2-Diphenyl-3,5-dioxo-4-(2-phenylsulfinyläthyl)-pyrazolidin im Verhältnis von 10:1 bis 1:10 kombiniert und den bei der Herstellung von Arzneimitteln üblichen Träger- und/oder Hilfsstoffen zu Tabletten, Dragees, Pulver für Briefe und Kapseln formuliert wird. Die erfindungsgemäßen neuen Präparate werden vorzugsweise peroral in den oben angeführten Dosen verabreicht.

Die Herstellung von Tabletten und Dragees, Kapseln erfolgt in an sich bekannter Weise, beispielsweise werden die Tabletten durch unmittelbares Verpressen eines Gemisches der Wirk- und Hilfsstoffe hergestellt und gegebenenfalls nachträglich mit einem im Magen- und Darm verträglichen Film überzogen, bei der Herstellung der Kapseln wird erst die Pulvermischung und der Kern mit Überzug getrennt hergestellt und dann auf einer handelsüblichen Kapselabfüllmaschine abgefüllt.

Die folgenden Beispiele erläutern die Erfindung ohne sie zu beschränken:

## BEISPIEL 1

Dragees mit 50 mg Dipyridamol und 50 mg Sulfinpyrazon

Ein Drageekern enthält:

| | | | |
|---|---|---|---|
| Dipyridamol | 50,0 | mg | Wirkstoff |
| Sulfinpyrazon | 50,0 | mg | Wirkstoff |
| Milchzucker | 187,0 | mg | Hilfsstoff |
| Maisstärke | 105,0 | mg | Hilfsstoff |
| Gelatine | 6,0 | mg | Hilfsstoff |
| Magnesiumstearat | 2,0 | mg | Antiadhäsionsmittel |
| | 400,0 | mg | |

Die Wirkstoffe werden zusammen mit Milchzucker und Stärke gemischt und die Mischung gleichmäßig mit einer wäßrigen Gelatinelösung befeuchtet. Die feuchte Masse wird auf max. 2 mm gesiebt, getrocknet und nach nochmaligem Sieben mit dem Schmiermittel vermischt. Aus der so hergestellten preßfertigen Mischung wurden Tabletten von 400 mg gepreßt.

Drageekernmaße:
Gewicht:          ca. 400 mg

Form              rund, bikonvex

Durchmesser       11 mm

Dragierung:
Die Kerne werden mit einer üblichen Zuckerdragiersuspension auf 500 mg und anschließend mit Zuckersirup auf 530 mg/Dragee dragiert.

3

# 0 000 774

BEISPIEL 2

Kapseln mit 50 mg Dipyridamol und 50 mg Sulfinpyrazon

| Kapselhülle: | Hartgelatine-Kapsel Größe 0 | | |
|---|---|---|---|
| Kapselinhaltsstoffe: | | | |
| Pulvermischung: | | | |
| Sulfinpyrazon | 50,0 mg | Wirkstoff |
| Maisstärke | ca. 158,0 mg | Füllmittel |
| Milchzucker plv. | ca. 90,0 mg | Füllmittel |
| Magnesiumstearat | 2,0 mg | Antiadhäsionsmittel |
| | 300,0 mg | |
| Kern $\phi$ 6 mm: | | |
| Dipyridamol | 50,0 mg | Wirkstoff |
| Kollidon 25$^R$ (Polyvinylpyrrolidon) | 2,5 mg | Bindemittel |
| Formaldehydgelatine | 6,5 mg | Sprengmittel |
| Magnesiumstearat | 1,0 mg | Antiadhäsionsmittel |
| Milchzucker | 25,0 mg | |
| Überzug: | | |
| Talkum | ca. 13,4 mg | Dragierhilfsstoff |
| Zucker | ca. 4,2 mg | Dragierhilfsstoff |
| Gummi arabicum | ca. 2,4 mg | Dragierhilfsstoff |
| | ca. 105,0 mg | |
| Gesamtfüllgewicht: | Pulver/Kern ca. 405 mg. | |

## Patentansprüche

1. Antithrombotische Arzneimittelkombination, dadurch gekennzeichnet, daß sie als Wirkstoff 2,6 - Bis(diäthanolamino) - 4,8 - dipiperidino - pyrimido[5,4 - d]pyrimidin, 1,2 - Diphenyl - 3,5 - dioxo- 4 - (2 - phenylsulfinyläthyl) - pyrazolidin und die üblichen Träger und/oder Hilfsstoffe enthält.

2. Arzneimittelkombination nach Anspruch 1, dadurch gekennzeichnet, daß das Gewichtsverhältnis von 2,6 - Bis(diäthanolamino) - 4,8 - dipiperidino - pyrimido[5,4 - d]pyrimidin zu 1,2 - Diphenyl- 3,5 - dioxo - 4 - (2 - phenylsulfinyläthyl) - pyrazolidin 10:1 bis 1:10 beträgt.

3. Arzneimittelkombination gemäß Anspruch 1 und 2, dadurch gekennzeichnet, daß das Verhaltnis der beiden Wirkstoffe 1:1 beträgt.

4. Verfahren zur Herstellung einer antithrombotischen Arzneimittelkombination, dadurch gekennzeichnet, daß man 2,6 - Bis(diäthanolamino) - 4,8 - dipiperidino - pyrimido[5,4 - d]pyrimidin und 1,2 - Diphenyl - 3,5 - dioxo - 4 - (2 - phenylsulfinyläthyl) - pyrazolidin im Verhältnis von 10:1 bis 1:10 kombiniert und mit den üblichen Träger- und/oder Hilfsstoffen zu Dragees, Tabletten, Kapseln oder Pulverbriefchen formuliert.

4

**Claims**

1. Antithrombotic pharmaceutical combination, characterised in that it contains as active ingredient 2,6 - bis(diethanolamino) - 4,8 - dipiperidino - pyrimido[5,4 - d]pyrimidine, 1,2 - diphenyl- 3,5 - dioxo - 4 - (2 - phenylsulphinylethyl) - pyrazolidine and conventional carriers and/or excipients.

2. Pharmaceutical combination according to claim 1, characterised in that the weight ratio of 2,6 - bis(diethanolamino) - 4,8 - dipiperidino - pyrimido[5,4 - d] - pyrimidine to 1,2 - diphenyl - 3,5- dioxo - 4 - (2 - phenylsulphinylethyl) - pyrazolidine is 10:1 to 1:10.

3. Pharmaceutical combination according to claims 1 and 2, characterised in that the ratio of the two active substances is 1:1.

4. Process for the preparation of an antithrombotic pharmaceutical combination, characterised in that 2,6 - bis(diethanolamino) - 4,8 - dipiperidino - pyrimido[5,4 - d] - pyrimidine and 1,2 - diphenyl- 3,5 - dioxo - 4 - (2 - phenylsulphinylethyl) - pyrazolidine are combined in a ratio of 10:1 to 1:10 and are formulated with conventional carriers and/or excipients into coated tablets, tablets, capsules or powder envelopes.

**Revendications**

1. Combinaison médicamenteuse antithrombotique, caractérisée en ce qu'elle renferme, en tant que substance active de la 2,6 - bis(diéthanolamino) - 4,8 - dipipéridino - pyrimido[5,4 - d]- pyrimidine, de la 1,2 - diphényl - 3,5 - dioxo - 4 - (2 - phénylsulfinyléthyl) - pyrazolidine et les excipients et/ou adjuvants habituels.

2. Combinaison médicamenteuse selon la revendication 1, caractérisée en ce que le rapport en poids de la 2,6 - bis(diéthanolamino) - 4,8 - dipipéridino - pyramido[5,4 - d]pyrimidine à la 1,2- diphényl - 3,5 - dioxo - 4 - (2 - phénylsulfinyléthyl) - pyrazolidine est de 10:1 à 1:10.

3. Combinaison médicamenteuse selon la revendication 1 ou 2, caractérisée en ce que le rapport des deux substances actives est de 1:1.

4. Procédé de préparation d'une combinaison médicamenteuse antithrombotique, caractérisé en ce que l'on combine de la 2,6 - bis(diéthanolamino) - 4,8 - dipipéridino - pyrimido[5,4 - d]pyrimidine et de la 1,2 - diphényl - 3,5 - dioxo - 4 - (2 - phénylsulfinyléthyl) - pyrazolidine dans le rapport de 10:1 à 1:10 et en ce qu'on formule avec les excipients et/ou adjuvants habituels, pour donner des dragées, des comprimés, des capsules ou des sachets de poudre.